# EUROPEAN PATENT APPLICATION

(11) **EP 1 371 726 A1**
(43) Date of publication of application: **17.12.2003**
(21) Application number: 02077353.7
(22) Date of filing: 14.06.2002
(51) Int. Cl.: C12N 15/10, C12Q 1/68

(54) **Method for amplifying RNA**

(71) Applicant: Rijksuniversiteit Groningen, 9712 CP Groningen (NL)
(72) Inventor: Boddeke, Erik Hendrikus Wilhelmus Gerardus Maria, 9753 AX Haren (NL); Biber, Knut Peter Heinrich, 9961 PD Mensingeweer (NL)
(74) Representative: Prins, Adrianus Willem

(57) **Abstract**

The invention relates to the field of enzymatic gene amplification, and particularly to the provision of multigene expression profiles derived from messenger RNA. Then invention provides a method for amplifying RNA from a sample, preferably for obtaining an expression profile of cellular RNA, comprising the step of amplifying (cellular) RNA by contacting said RNA with an a first amplification primer, an RNA-dependent polymerase and nucleotides to synthesize a copy nucleic acid from said RNA, wherein said first amplification primer comprises a 3'-fragment capable of hybridizing with a poly-A tail of mRNA and said RNA-dependent polymerase is capable of providing an extension to said first strand copy nucleic acid allowing hybridisation with a second amplification primer.

## Description

The invention relates to the field of enzymatic gene amplification, and particularly to the provision of multigene expression profiles derived from messenger RNA.

In many applications of biotechnology, such as studying the regulation or expression of a gene or genes of hosts or pathogens that are associated with manifestations of normal physiology or pathophysiology of said hosts (in particular of multicellular organisms) it is important to study the expression of said gene or genes present in a particular single cell or related cell population. In particular for multiple genes, such studies amount to providing a profile of the genes expressed in said cell in a particular phase in its development. Although it would also suffice to provide an insight in the identity and number of (active) proteins present in said cell or cells, such a profile is in general made by determining the relative abundance of mRNA or mRNA species derived from one or more genes present in that cell or cell population (see for example Sarkar et al., Access to a messenger RNA sequence or its protein product is not limited by tissue or species specificity. Science 244:331,1989)

The availability of high quality genetic material (RNA) is essential for modern cloning- and expression studies in research institutes, hospitals and drug companies. Since isolation of quantities of high quality RNA, sufficient for analysis is complicated, a number of biotech companies now offer various RNA products. Generally, these RNA products are isolated from tissues like liver, skin, thymus, heart, brain etc. Genetic analysis performed with these products is insensitive and inaccurate.

For example, in general not advisable to use well known reverse-transcriptase polymerase chain reaction (RT-PCR) techniques for determining the relative abundance of mRNA or mRNA species derived from one or more genes present. For one, PCR works best with relatively short nucleic acid fragments, longer fragments are not as readily amplified as shorter ones, giving raise to misrepresentation of the abundance of short mRNA species over longer ones. Also of note is that the commonly used Taq polymerase introduces a too high level of erroneous bases per round of amplification. These technological problems limit the usefulness of PCR in the provision of a, in particular multigene, expression profile derived from messenger RNA.

Early applications (such as van Gelder et al., Amplified RNA synthesized from limited quantities of heterogeneous cDNA, PNAS 87:1663, 1990 and Eberwine et al., Analysis of gene expression in single live neurons. PNAS 89:3010, 1992), of (single)cell-expression-profiling not using common PCR date but providing cDNA's from mRNA and amplifying RNA from said cDNA with aid of an RNA polymerase date from the early 1990's. It is said that these methods provide a truer reflection of the relative abundancies of the mRNA species present, in particular to the amplification of a polyA-mRNA population, derived from cells or even within a single cell. Some of these insights were further detailed in among others US patents 5,515,545, 5,545,522, 5,716,785, 5,891,636, 5,958,688 and 6,291,170.

These insights are well applicable to provide for specific amplification of distinct RNA fragments when at least the two terminal parts of the sequence of the RNA-species to be amplified are essentially known and more-or-less specific primers can be designed, but urge to resort to use random primers, such as random hexanucleotides when (sub)sets of various mRNA species need be amplified of which the relevant sequences are not known, as is most often the case when an expression profile of a given cell needs to be established.

Considering that such random primers will by default likely not hybridize to, or even close to, the most terminal end of a specific mRNA species, again these methods fall short where nearly-full-length amplification of all or a large part of a cellular mRNA population is desired.

The invention provides technology allowing essentially complete isolation of RNA from one single cell followed by enzymatic amplification. This technology produces total high quality RNA from single cells in quantities, which are sufficient for conventional genetic analysis. The precise and sensitive genetic analysis possible using this technology offers great advantages compared to existing products.

The invention provides a method for obtaining an expression profile of cellular, preferably messenger, RNA wherein the desired for nearly-full-length amplification of all or a large part of a cellular mRNA population is better met.

High quality RNA is essential for modern cloning and gene expression studies. Since isolation of quantities of high quality RNA, sufficient for analysis is complicated, a number of biotech companies now offer various RNA products. Generally, these RNA products are isolated from tissues like liver, skin, thymus, heart, brain etc. Genetic analysis performed with these products is insensitive and inaccurate. Organ tissue, however, consist of many different cell types each displaying a specific RNA expression profile. RNA extracts from whole tissue therefore represent mixtures of expression profiles of many different cell types, which have a limited value for gene expression analysis.
The technology described here offers a solution for these problems: The RNA products provided by here are produced from single, and preferably histologically characterized cells. Due to the specific production procedure several batches of essentially identical RNA samples can be produced from a single cell. This allows the composition of an extended RNA library containing a large number of cells isolated from healthy and diseased tissues.

In particular, the invention provides a method for amplifying RNA from a sample, preferably for obtaining an expression profile of cellular RNA,
comprising the step of: a) amplifying RNA, preferably cellular RNA, most preferably RNA obtained from a single cell, by contacting said RNA with an a first amplification primer, an RNA-dependent polymerase and nucleotides to synthesize a first strand copy nucleic acid (cDNA) from said RNA. It is preferred that said polymerase is at least a DNA polymerase, so that said first strand copy nucleic acid is cDNA. It is furthermore preferred that said first amplification primer comprises a 3'-fragment capable of hybridizing with a poly-A tail of mRNA (in general a poly A-tail comprises at least 10 adenosides) and said RNA-dependent polymerase is capable of providing an extension to said first strand copy nucleic acid (preferably of at least two distinct nucleotides) allowing hybridisation with a second amplification primer.

For the priming of the second strand synthesis in the first amplification round (and the priming of the third strand synthesis of the second amplification round) random hexamers may be used. The use of the random hexamers in general however leads to a 3'-bias in the amplified material and loss of information of full-length genes. It is preferred to generate full-length copy nucleic acid derived from full-length mRNA derived from one cell. In one embodiment of the invention, it is provided to include a so-called template switch primer for the second and/or fourth strand synthesis. In one such embodiment, said template switch primer is preferably capable of selectively interacting with a 5'-structure of a full-length mRNA-cDNA intermediate generated during above identified first strand synthesis, wherein said template switch primer interacts with said 5'- structure (be it a 5'-CAP mRNA structure or plain 3'-added nucleotides to said first strand copy nucleic acid) and wherein said template switch primer is capable of additional first round synthesis such that a sequence complementary to said template switch primer is further incorporated at the 3'-end of said first strand copy nucleic acid.

Preferably, such a template switch primer recognizes two or more preferably three additional C residues that are added by reverse transcriptase at the 5'-end of mRNA. Using a second and/or fourth amplification primer with identical sequence to said template switch primer, the second and/or fourth strand synthesis will therefore start at the 5'-end of the generated copy nucleic acid. A preferred sequence of such a template switch primer is 5'-AAGCAGTGGTATCAACGCAGAGCGGG-3'. Herein A, G, C, T and U stand for the bases adenine, guanine, cytosine (found in both DNA and RNA) thymine (found only in DNA) and uracil (found only in RNA). In a preferred embodiment the invention provides a method wherein said 3'-fragment capable of hybridizing with a poly-A tail of mRNA comprises an oligo(dT) fragment. Such fragments can be of various length, the optimal number of thymidines lays between 5-50, preferably 10-35.

When as a first strand copy nucleic aid cDNA is preferred, it is preferred that said RNA-dependent polymerase comprises a DNA-polymerase such as a reverse transcriptase. It is preferred that said reverse transcriptase is capable of providing said extension with at least two cytosines.

Furthermore, the invention provides a method according wherein said first amplification primer additionally comprises an RNA polymerase promotor. One aim of method is to make RNA expression profiling from an RNA-population from single cells, thereby as truthfully reflecting the relative numbers of RNA-species present. Using T7 RNA polymerase it is possible to transcribe this cDNA into significantly more RNA. The amplification is linear that means the resulting RNA reflects the original RNA population. It is preferred that said RNA polymerase comprises T7 polymerase, however, SP6 or T3 polymerase may for example also be used

Furthermore, the invention provides a method comprising the step of:
b) amplifying said first strand copy nucleic acid, in the examples provided herein cDNA is used by contacting said nucleic acid with a second amplification primer, a (in the case of cDNA preferably specific DNA) polymerase and nucleotides to synthesize a second strand copy nucleic acid (such as cDNA), wherein said second amplification primer comprises a 3'-fragment capable of hybridizing with said extension to said first strand copy nucleic acid, said method preferably also comprising the step of:
c) amplifying said second strand copy nucleic by contacting said nucleic acid with a suitable (T7-RNA polymerase is very suitable to make cRNA) polymerase and nucleotides to synthesize a third strand copy nucleic acid, wherein said third amplification primer comprises a 3'-fragment capable of hybridizing with said 3'-fragment of said first amplification primer.

It is preferred that said third amplification primer comprises an oligo(dA) fragment, preferably linked to an RNA polymerase promotor, such as the T7-RNA polymerase promotor identified herein.

Furthermore, the invention provides a method comprising the step of:
d) amplifying said third strand copy nucleic acid by contacting said nucleic acid with said second amplification primer, a polymerase and nucleotides to synthesize a fourth strand copy nucleic acid, preferably cDNA.

First and second strand copy nucleic acid are preferably provided in a first amplification round, third and fourth strand copy nucleic acid preferably in a second amplification round, as explained in the detailed description herein In a further preferred embodiment, a method is provided comprising enriching said cellular RNA from said sample, for example by contacting said cellular RNA with said first amplification primer wherein said primer is provided with means allowing binding to a solid phase. Such a solid phase can be manifold, it is herein preferred to provided said primer with biotin and said solid phase with streptavidin. It is especially useful when said solid phase comprises a magnetic bead. The method provided is preferably used to synthesize bead-coupled cDNA with a T7 RNA polymerase site derived from the mRNA of a single cell. Use of magnetic streptavidin beads and 5'-biotin labelled primers is for example provided to prime first strand synthesis. The use of magnetic beads allows a rapid and reliable removal of the nucleic acids of buffer solutions and minimizes the time and chance of loosing material compared to standard precipitation steps. Such rapid and reliable removal is especially preferred when said cellular RNA is derived from a single cell. It is preferred to derived said RNA from a cell selected for having defined physiology, pathology or pathophysiology.

The invention also provides amplified nucleic acid (copy nucleic acid, be it cRNA or cDNA) obtained with a method according to the invention. In the detailed description, such amplified nucleic acid comprising RNA is provided after the first and second rounds of amplification.

The invention also provides a method for providing an expression profile comprising use of a nucleic acid according to the invention, preferably wherein said profile is multigenic. An RNA expression profile a provide gives a comparison of the abundances of at least two RNA's to each other. A multigenic RNA expression profile allows the comparison of the relative abundance of each of a multitude of mRNA's derived from the RNA population of a cell or tissue. There are different ways to look at RNA expression profiles, the simplest one is to isolate RNA by conventional methods and make Northenblot analysis or RT-PCR for the RNA's of interest. More complex ways to look at expression profiles involve reverse northern blotting, RNase Protection Assay, SAGE or gene arrays. All these techniques include the preparation of high quality RNA, preferably for example at least 0.5-1microgram. With reverse northern blotting or RNase protection assay and gene arrays the RNA is directly labeled and hybridized to a target blot or the RNA is modified by hybridization with an anti sense RNA probe or transcription into cDNA prior hybridization. The results of these techniques show bands or spots that indicate the abundance of a given RNA that can be compared with the abundance of another RNA. Expression profiling by SAGE is different. Here it is also possible to investigate the expression of unknown RNA's. This technique includes the transcription of RNA into cDNA and the digestion of the cDNA with distinct restriction enzymes. The resulting fragments are cloned into vectors and sequenced. The expression profile is a lot of sequences and the abundance of a given RNA is calculated by the abundance of its sequence fragments in the total pool of sequences.

### Figure legends

### Figure 1

The messenger RNA (mRNA) from one cell is sucked in a glass micro pipette. Characteristically mRNA contains a poly-A tail which in the next step is recognized and hybridized by a poly-T-T7 primer. In addition to the poly-T the primer also contains a recognition site for the enzyme T7 polymerase. This primer is the starting point of the next reaction step, the reverse transcription. Using a reverse transcriptase enzyme cDNA is produced starting reverse transcription from the primer. Subsequently, DNA that is complementary to the cDNA is produced using the enzyme DNA polymerase thus generating double string DNA. The location of the poly-T-T7 primer now represents a starting site for T7 polymerase. This enzyme now generates new RNA molecules that are complementary to the original RNA. This RNA can be used for a second amplification round.

### Figure 2

Magnetic beads may be used to bind many types of molecules thus constituting a versatile solid phase. For the described experiments the beads contain streptavidine molecules attached to their surface which specifically recognize and biotine molecules. By coupling the poly-T-T7 primer to a biotine molecule, the primer can thus be attached to the beads. Using a magnet the beads and the attached reaction products can now easily be separated from solutions thus facilitating the required purification steps.

### Figure 3

Combining the amplification technique with primers coupled to magnetic beads accelerates the purification of the reaction products after each enzyme reaction. Furthermore, the loss of reaction product is strongly reduced and less degradation of RNA occurs.

### Detailed description

After sequencing the human genome estimations of the number of human genes range from 35.000 to 70.000. Soon most of these genes will be mapped and sequenced and this will end the era of description and discovery of novel human genes. The new biological challenge will be the correlation of gene expression patterns with cell function/physiology. Accordingly, analysis of single cell RNA expression profiles should be considered as "fingerprints" of (patho)physiological processes occurring in cells.
The amount of RNA that can be extracted from one cell (1-10 picogram) is far too low for conventional RNA expression analysis techniques using Northern blotting, cDNA arrays or I-SAGE which need a critical amount of approximately 1 µgram mRNA (this amounts to 1 million cells). Accordingly, for conventional RNA isolation tissue samples are used that contain several millions of cells. The major problem of this strategy, however, is the large variety of cell types present in tissues, representing different mRNA expression profiles. This implies that RNA isolated from tissue represents a mixture of expression profiles; it will thus be impossible to obtain from a tissue sample information on the expression profile of one specific cell type. Specific changes in gene expression in one cell type underlying (patho)physiological signaling or resulting from drug treatment cannot be detected either in tissue samples. In brief: the specificity and sensitivity of cellular gene expression still form a major obstacle within genetic research.
On the other hand a rapid development of gene detection technology including differential display, SAGE and cDNA array techniques has occurred. Particularly due to the fast development of cDNA array technology the analysis of gene expression has gained much interest in medical clinical and pharmaceutical research.
According an urgent need for improvement of RNA amplification technology from single cells is required. We have developed new technology allowing complete isolation of RNA from one single cell followed by enzymatic amplification. This technology produces total high quality RNA from single cells in quantities, which are sufficient for conventional genetic analysis. This technology will allow research groups, hospitals and drug companies to perform single cell RNA expression analysis on a routine basis, providing versatile tools for pathological screening, drug target finding, pharmacogenomics and functional genomics.

We have developed technology to produce **So**lid **P**hase **Si**ngle **C**ell **cDNA** using a **T7** promoter **(*T7-SopSic-cDNA*)** allowing us to reproduce by linear RNA amplification the total RNA expression profile of single cells in quantities sufficient to perform conventional gene expression analysis. Since ***T7-SopSic-cDNA*** is coupled to a "solid phase" (magnetic beads) it is feasible to reproduce the expression profile a single cell several times. Accordingly, ***T7-SopSic-cDNA*** is a unique and reliable tool for RNA-based research. Linear RNA amplification is a technology which is perfectly suited for analysis of RNA expression in individual cells. The technology is based on amplification of the total RNA from one single cell such that all RNAs expressed by this cell can be detected and analyzed. The conventional amplification technique is very complex and time-consuming and has for these reasons hardly been used. All reaction procedures are performed in solution such that the reaction products (e.g. the cDNA transcribed from the original RNA) have to be extracted several times from the solution. This method is not only time-consuming, due to these multiple extraction procedures a considerable amount of the reaction product is lost and the estimated time for one amplification is approximately one week.
We have developed an alternative strategy where the oligo- (dT)-T7 primer and thus the reaction product are coupled to magnetic beads. This means that the synthesis of the cDNA, - i.e. the matrix for RNA amplification - is performed at the solid phase. Due to this adaptation the extraction procedures are performed much more efficient such that the time required for one amplification is only two days and the yield of reaction product is much higher. Accordingly, the new method is more reliable and reproducible. As already described the availability of high quality RNA represents a serious obstacle for analysis of gene expression. Based on the development of ***T7-SopSic-cDNA*** it is now possible to extract and amplify RNA from single cells and small tissue samples. Since ***T7-SopSic-cDNA*** can be stored without limitation at - 20° C the product can be used several times. It is thus possible to establish a collection of ***T7-SopSic-cDNAs*** from a large number of well-characterized single cells. The following applications are examples of possible use:
RNA produced using ***T7-SopSic-cDNA*** can be used for cell-specific analysis of gene expression: instead of extraction of RNA from organ tissue the invention provides RNA isolated from specific cells from this organ. This will allow investigation of gene expression at the proper single cell level.
T7-SopSic-cDNA can be produced on a customized basis: this service can be performed on cell or tissue samples sent to us by customers.
RNA produced using ***T7-SopSic-cDNA*** can be used as a tool for drug target analysis: a catalogue of cells affected by disease and comparable healthy cells will be established.
A database of ***T7-SopSic-cDNA-based*** gene expression profiles generated using cDNA arrays will be established:

### Example 1

### Bead coupling

- Shake bead suspension vigorously and transfer calculated amount
- Wash twice with double volume of binding buffer (PBS containing 1 mol/l NaCl, DEPC treated)
- Resuspend to final concentration (20 mg/ml)
   Add biotin-labeled oligonucleotide: oligod(t)5'-Biotin- 
AAACGACGGCCAGTGAATTG
TAATACGACTCACTATAGGCGCTTTTTTTTTTTTTTTTTTTTTTTT-3`
 if it is the first amplification round. Use oligod(a)5'-Biotin- 
AAACGACGGCCAGTGAATTG
TAATACGACTCACTATAGGCGCAAAAAAAAAAAAAAAAAAAAAAAA-3`
 if it is the second amplification round.
- for maximal binding final concentration oligonucleotides should be 400-500 pmol per mg streptavidin beads, which will lead to a oligonucleotide concentration coupled to beads of 200pmol/mg beads.
- Incubate at room temperature, gently rotating for 1 hr
- Wash 3 times with double volume buffer (PBS or Tris-buffer) and resuspend in appropriate storage buffer (PBS or Tris-buffer)

### Single cell RNA preparation

Wash 10ul of the oligonucleotide coupled magnetic beads 2 times with 100ul reverse transcription buffer (RT) (see below) and resuspend in 45ul RT buffer.

Living cultured cells or cells in living tissue slices will be harvested with a patch pipette filled with 5ul RT buffer (see below). Add the content of the pipette to the 45ul oligonucleotide coupled magnetic beads (see below). Incubate for 15 min at room temperature in order to hybridize the mRNA to the oligonucleotides and start reverse transcription.

The RNA from cells in fixed material is harvested by cutting out single cells with laser dissection microscopy and lysis of the cells in 5ul lysis buffer (see below).

The lysis buffer is added to 45ul oligonucleotide coupled magnetic beads and incubate for 15 min at room temperature in order to hybridize the mRNA to the oligonucleotides. Wash the beads 2 times with 1x reverse transcription buffer and start reverse transcription.

### Reverse Transcriptase

Make reverse transcriptase mastermix (50ul per reaction)
- 31.5 µl dH2O
- 5 µl 10X first strand buffer
- 4 µl 100mM DTT (final conc. = 8mM)
- 4 µl 2.5 mM dNTPs (final conc. = 200µM)
- 0.5 µl Rnasin
put the beads in the magnetic stand and remove the 50ul RT buffer
add 50ul of the RT mastermix to the beads incubate for 15 minutes at 37°C
add 0.2 µl Reverse transcriptase per reaction, quickly to prevent cooling down incubate for 1.5 hrs at 42°C
incubate 2 minutes at 90°C
put immediately on ice

10X Reverse transcriptase Buffer (RT), pH = 8.3
500 mM Tris-base
1.2 M KCL
100 mM MgCl₂
0.5 mM NaPPi

### Second strand synthesis

Wash beads from reverse transcriptase 2 times in 100ul ice-cold 1x second strand buffer (SSB) (see below).

Prepare mastermix for second strand synthesis, 50ul per reaction.
- 33.5 µl dH2O
- 5 µl 10X SSB
- 5 µl 2.5mM dNTPs (final conc. = 250µM)
- 4 µl DTT (final conc. = 8mM)
- 1 µl random hexamers (100uM)
- 0.5 µl Rnasin
- 0.5 µl Klenow
- 0.5 µl T4 DNA polymerase
30 minutes at 14°C
30 minutes at 37°C
Preferably proceed with aRNA transcription, if not possible then store beads at 4°C

10X Second Strand Buffer (SSB), pH = 7.4
1 M Tris-base
200 mM KCL
100 mM MgCl₂
400 mM (NH₄)₂SO₄

### RNA amplification

Wash beads from second strand synthesis 2 times in 100ul 1x RNA amplification buffer (see below).

Mastermix: 40 µl total
- 28.3 µl dH2O
- 4 µl 10X amplification buffer
- 3.2 µl DTT
- 2 µl NTP mix
- 2.0 µl T7 polymerase
- 0.5 µl Rnasin
incubate 6 hours at 37°C,

put beads into the magnetic stand, harvest the RNA and pipette the RNA in a new Rnàse free reaction cup.
RNA will be precipitated as follows
- wash the beads with dH2O (as much as to make a total volume of 90 µl) and add the solution to the RNA
- Wash beads 2x with 100ul storage buffer and store at 4°C.
- add 10 µl 3M NaAc (pH=5.2)
- add 5 µg tRNA (stock= 0.1µg/µl, so 5 µl)
- add 300 µl 100% EtOH
- precipitate on dry-ice until viscous, preferably overnight
- centrifuge 15 min. max. speed, 4°C
- remove supernatant and dry pellet thoroughly
Dissolve RNA pellet in 10ul water and use the RNA in subsequent second amplification round.

10X aRNA Amplification Buffer, pH = 7.5
400 mM Tris-base
70 mM MgCl₂
100 mM NaCl
2 mM Spermidine

### Other buffers used

Lysis Buffer
100 mM Tris-HCL, pH 7.5
500mM LiCl
10 mM EDTA, pH 8.0
5 mM dithiothreitol (DTT)
1% LiDS

Storage Buffer
250 mM Tris-HCL, pH 7.0
20 mM EDTA
0.1% Tween-20
0.02% Sodium azide

### Example 2

### Bead coupling

- Shake bead suspension vigorously and transfer calculated amount
- Wash twice with double volume of binding buffer (PBS containing 1 mol/l NaCl, DEPC treated)
- Resuspend to final concentration (20 mg/ml)
   Add biotin-labeled oligonucleotide: oligod(t)5'-Biotin- 
AAACGACGGCCAGTGAATTG
TAATACGACTCACTATAGGCGCTTTTTTTTTTTTTTTTTTTTTTTTTTTTTTTTTTTTTTT-3'
 if it is the first amplification round. Use oligod(a)5'-Biotin- 
AAACGACGGCCAGTGAATTG
TAATACGACTCACTATAGGCGCAAAAAAAAAAAAAAAAAAAAAAAA-3`
 if it is the second amplification round.
- for maximal binding final concentration oligonucleotides should be 400-500 pmol per mg streptavidin beads, which will lead to a oligonucleotide concentration coupled to beads of 200pmol/mg beads.
- Incubate at room temperature, gently rotating for 1 hr
- Wash 3 times with double volume buffe (PBS or Tris-buffer) and resuspend in appropriate storage buffer (PBS or Tris-buffer)

### Single cell RNA preparation

Wash 10ul of the oligonucleotide coupled magnetic beads 2 times with 100ul reverse transcription buffer (RT) (see below) and resuspend in 45ul RT buffer.

Living cultured cells or cells in living tissue slices will be harvested with a patch pipette filled with 5ul RT buffer (see below). Add the content of the pipette to the 45ul oligonucleotide coupled magnetic beads (see below). Incubate for 15 min at room temperature in order to hybridize the mRNA to the oligonucleotides and start reverse transcription.

The RNA from cells in fixed material is harvested by cutting out single cells with laser dissection microscopy and lysis of the cells in 5ul lysis buffer (see below).

The lysis buffer is added to 45ul oligonucleotide coupled magnetic beads and incubate for 15 min at room temperature in order to hybridize the mRNA to the oligonucleotides. Wash the beads 2 times with 1x reverse transcription buffer and start reverse transcription.

### Reverse Transcriptase

Make reverse transcriptase mastermix (50ul per reaction)
- 31.5 µl dH2O
- 5 µl 10X first strand buffer
- 4 µl 100mM DTT (final conc. = 8mM)
- 4 µl 2.5 mM dNTPs (final conc. = 200µM)
- 0.5 µl Rnasin
- 5ul of template switch primer 5'-AAGCAGTGGTATCAACGCAGAGCGGG-3' (100uM)

put the beads in the magnetic stand and remove the 50ul RT buffer
add 50ul of the RT mastermix to the beads incubate for 15 minutes at 37°C
add 0.2 µl Reverse transcriptase per reaction, quickly to prevent cooling down incubate for 1.5 hrs at 42°C
incubate 2 minutes at 90°C
put immediately on ice

10X Reverse transcriptase Buffer CRT), pH = 8.3
500 mM Tris-base
1.2 M KCL
100 mM MgCl₂
0.5 mM NaPPi

### Second strand synthesis

Wash beads from reverse transcriptase 2 times in 100ul ice-cold 1x second strand buffer (SSB) (see below).

Prepare mastermix for second strand synthesis, 50ul per reaction.
- 34.5 µl dH2O
- 5µl 10X SSB
- 5 µl 2.5mM dNTPs (final conc. = 250µM)
- 4 µl DTT (final conc. = 8mM)
- 0.5 µl Rnasin
- 0.5 µl Klenow
- 0.5 µl T4 DNA polymerase
30 minutes at 14°C
30 minutes at 37°C
Preferably proceed with aRNA transcription, if not possible then store beads at 4°C

10X Second Strand Buffer (SSB), pH = 7.4
1 M Tris-base
200 mM KCL
100 mM MgCl₂
400 mM (NH₄)₂SO₄

### RNA amplification

Wash beads from second strand synthesis 2 times in 100ul 1x RNA amplification buffer (see below).

Mastermix: 40 µl total
- 28.3 µl dH2O
- 4 µl 10X amplification buffer
- 3.2 µl DTT
- 2 µl NTP mix
- 2.0 µl T7 polymerase
- 0.5 µl Rnasin
incubate 6 hours at 37°C,

put beads into the magnetic stand, harvest the RNA and pipette the RNA in a new Rnàse free reaction cup.

RNA will be precipitated as follows
- wash the beads with dH2O (as much as to make a total volume of 90 µl) and add the solution to the RNA
- Wash beads 2x with 100ul storage buffer and store at 4°C.
- add 10 µl 3M NaAc (pH=5.2)
- add 5 µg tRNA (stock= 0.1µg/µl, so 5 µl)
- add 300 µl 100% EtOH
- precipitate on dry-ice until viscous, preferably overnight
- centrifuge 15 min. max. speed, 4°C
- remove supernatant and dry pellet thoroughly
Dissolve RNA pellet in 10ul water and use the RNA in subsequent second amplification round.

10X aRNA Amplification Buffer, pH = 7.5
400 mM Tris-base
70 mM MgCl₂
100 mM NaCl
2 mM Spermidine

### Other buffers used

Lysis Buffer
100 mM Tris-HCL, pH 7.5
500mM LiCl
10 mM EDTA, pH 8.0
5 mM dithiothreitol (DTT)
1% LiDS

Storage Buffer
250 mM Tris-HCL, pH 7.0
20 mM EDTA
0.1% Tween-20
**0.02% Sodium azide**

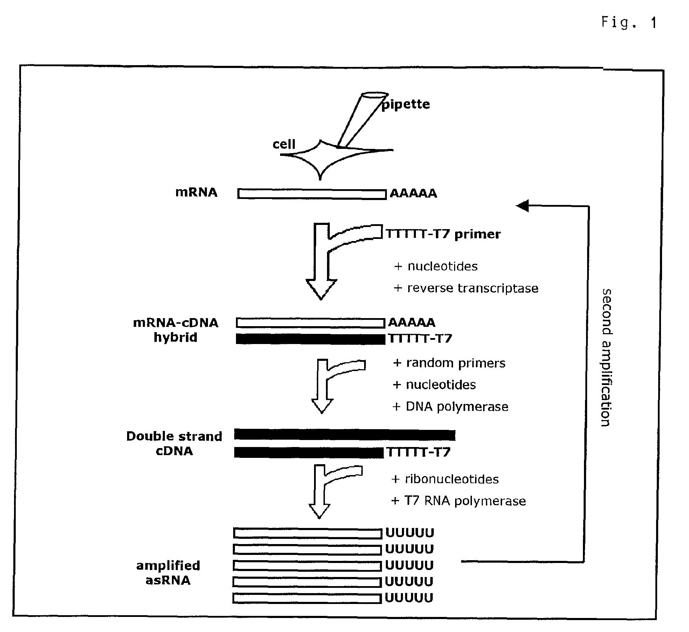

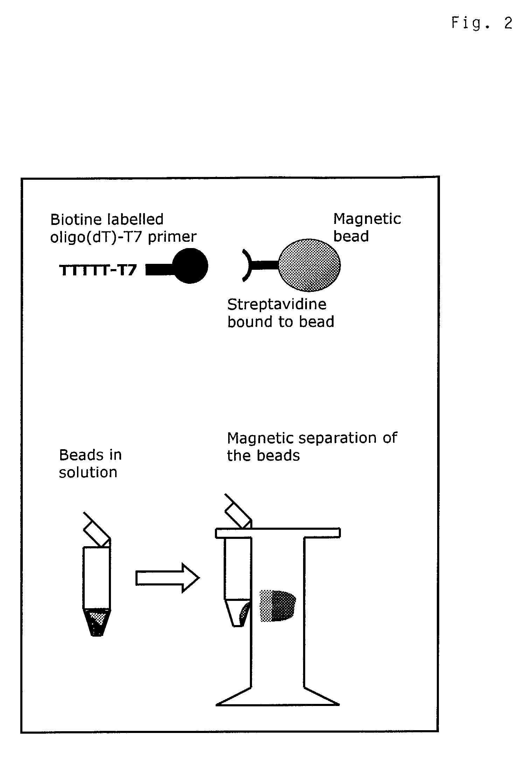

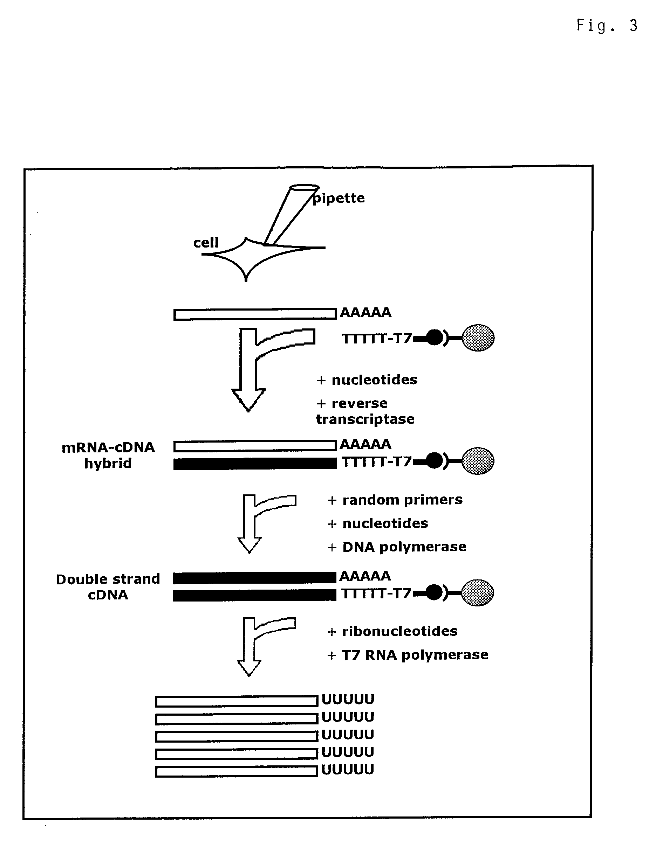

## Claims

1. A method for amplifying RNA from a sample comprising the step of:
a) amplifying RNA by contacting said RNA with an a first amplification primer, an RNA-dependent polymerase and nucleotides to synthesize a first strand copy nucleic acid from said RNA, wherein said first amplification primer comprises a 3'-fragment capable of hybridizing with a poly-A tail of mRNA and said RNA-dependent polymerase is capable of providing a nucleotide extension to said first strand copy nucleic acid said extension allowing hybridisation with a second amplification primer.

2. A method according to claim 1 wherein said 3'-fragment capable of hybridizing with a poly-A tail of mRNA comprises an oligo(dT) fragment.

3. A method according to claim 1 or 2 wherein said RNA-dependent polymerase comprises reverse transcriptase.

4. A method according to claim 3 wherein said reverse transcriptase is capable of providing said extension with at least two cytosines.

5. A method according to anyone of claims 1 to 4 wherein said first amplification primer additionally comprises an RNA polymerase promotor

6. A method according to claim 5 wherein said RNA polymerase comprises T7 polymerase.

7. A method according to anyone of claims 1 to 6 further comprising the step of:
b) amplifying said first strand copy nucleic acid by contacting said nucleic acid with a second amplification primer, a polymerase and nucleotides to synthesize a second strand copy nucleic acid, wherein said second amplification primer comprises a 3'-fragment capable of hybridizing with said extension to said first strand copy nucleic acid.

8. A method according to claim 7 further comprising the step of:
c) amplifying said second strand copy nucleic acid by contacting said nucleic acid with a polymerase and nucleotides to synthesize a third strand copy nucleic acid, wherein said third amplification primer comprises a 3'-fragment capable of hybridizing with said 3'-fragment of said first amplification primer.

9. A method according to claim 8 wherein said third amplification primer comprises an oligo(dA) fragment.

10. A method according to claim 8 or 9 further comprising the step of:
d) amplifying said third strand copy nucleic acid by contacting said nucleic acid with said second amplification primer, a polymerase and nucleotides to synthesize a fourth strand copy nucleic acid.

11. A method according to anyone of claims 1 to 10 further comprising enriching said cellular RNA from said sample.

12. A method according to claim 11 comprising contacting said cellular RNA with said first amplification primer wherein said primer is provided with means allowing binding to a solid phase.

13. A method according to claim 12 wherein said primer is provided with biotin and said solid phase is provided with streptavidin.

14. A method according to claim 12 or 13 wherein said solid phase comprises a magnetic bead.

15. A method according to anyone of claims 1 to 14 wherein said cellular RNA is derived from a single cell.

16. Amplified nucleic acid obtained with a method according to anyone of claim 1 to 15.

17. Amplified nucleic acid according to claim 16 comprising RNA.

18. A method for providing an expression profile comprising use of a nucleic acid according to claim 16 or 17.

19. A method according to claim 18 wherein said profile is multigenic.

20. An expression profile obtained with a method according to claim 18 or 19.
